(19) Europäisches Patentamt
European Patent Office
Office européen des brevets

(11) EP 1 163 905 A1

(12) **EUROPEAN PATENT APPLICATION**
published in accordance with Art. 158(3) EPC

(43) Date of publication:
**19.12.2001 Bulletin 2001/51**

(21) Application number: **99922593.1**

(22) Date of filing: **31.05.1999**

(51) Int Cl.[7]: **A61K 31/20**, A61K 47/10, A61K 47/30, A61K 7/48, A61K 35/60, A61K 35/78

(86) International application number:
**PCT/JP99/02883**

(87) International publication number:
**WO 00/32184 (08.06.2000 Gazette 2000/23)**

(84) Designated Contracting States:
**CH DE FR GB IT LI NL**

(30) Priority: **30.11.1998 JP 34048898**

(71) Applicants:
- **NOF CORPORATION**
  **Tokyo 150-0013 (JP)**
- **Ishihara, Kazuhiko**
  **Kodaira-shi, Tokyo 187-0022 (JP)**
- **Nakabayashi, Nobuo**
  **Matsudo-shi, Chiba 270-0021 (JP)**

(72) Inventors:
- **ISHIHARA, Kazuhiko**
  **Kodaira-shi, Tokyo 187-0022 (JP)**
- **NAKABAYASHI, Nobuo**
  **Matsudo-shi, Chiba 270-0021 (JP)**
- **SHIMADA, Kunio**
  **Toride-shi, Ibaraki 302-0015 (JP)**
- **TANAKA, Yukihisa**
  **Tsukuba-shi, Ibarari 305-0045 (JP)**
- **NAKANO, Yoshio**
  **Tsukuba-shi, Ibaraki 305-0045 (JP)**

(74) Representative: **Hallybone, Huw George et al**
**Carpmaels and Ransford, 43 Bloomsbury Square**
**London WC1A 2RA (GB)**

(54) **SKIN PREPARATIONS FOR EXTERNAL USE**

(57) Skin external preparation composition applicable to atopic dermatitis, etc. and exhibit little side effect, containing a natural oil and/or fat (A) containing an ω-3 unsaturated fatty acid and an ω-6 unsaturated fatty acid, the content of the ω-3 unsaturated fatty acid being 4 times or more the content of the ω-6 unsaturated fatty acid; a phosphorylcholine-like group containing polymer (B); and an antioxidant (C).

EP 1 163 905 A1

## Description

The present invention relates to a skin external preparation composition. More particularly, it relates to a skin external preparation composition applicable as a skin external preparation base in a drug or a cosmetic material to be applied to skin roughening, crazing, capped skin, dermatitis, chilblain, eczema, itching, sore, miliaria, insect bite and sting, atopic dermatitis, or the like.

In the present society, allergic diseases are increasing due to changes and deteriorations in living environments for people, changes in eating habits, stresses, and the like. Accordingly, skin disorders resulting from such diseases, particularly atopic dermatitis has shown a sharp increase in recent years. The atopic dermatitis occurs frequently and mostly during the infant period. However, patients beyond adolescence are on the increase in recent years, and their clinical histories are also long. Drugs such as an adrenal cortical hormone whereby high pharmacological effects can be expected are used in the treatment for the atopic dermatitis. However, the side effect resulting from such an adrenal cortical hormone drug becomes a concern, and a patient is exposed to a risk of the side effect during the treatment. Further, it is well known that a miserable rebound (the drastic exacerbation of the affected part occurring after stopping the use of the drug) is caused by stopping the use of the adrenal cortical hormone drug. In order to avoid such a side effect, a nonsteroidal anti-inflammatory drug or antihistamine not containing hormones such as an adrenal cortical hormone is used.

Incidentally, various proposals have been conventionally made as skin external preparations. Mention may be made of the following reports.

In Japanese Patent Laid-Open Publication No. Sho 61-221107, there is proposed a skin external preparation using a liver oil, and a hydroquinone glycoside such as hydroquinone-β-D-glucoside as a stabilizer. In Japanese Patent Laid-Open Publication No. Hei 3-279314, there is proposed a skin external preparation using a liver oil, and tranexamic acids as a stabilizer. In Japanese Patent Laid-Open Publication No. Hei 6-247852, there is disclosed that an external preparation in which vitamin E and squalene derived from in a shark liver oil are blended is effective for inflammatory dermatitis such as atopic dermatitis or contact dermatitis. In Japanese Patent Laid-Open Publication No. Hei 7-82162, there is disclosed that a skin external preparation using a hydrogenated liver oil obtained by hydrogenating the double bond of the unsaturated fatty acid contained in a liver oil.

Further, the following proposals have been made on a skin external preparation using an eicosapentaenoic acid (below, abbreviated as EPA), a docosahexaenoic acid (below, abbreviated as DHA), an arachidonic acid (below, abbreviated as ArA), and an α-linolenic acid (below, abbreviated as ALA).

In Japanese Patent Laid-Open Publication No.Hei 3-90022, the effect of EPA on allergic dermatitis such as atopic dermatitis is examined, and it is disclosed that excellent effects on the treatment of psoriasis or eczema can be obtained by using an EPA ethyl ester. In Japanese Patent Laid-Open Publication Nos.Hei 5-43456, 6-40887, 7-112913, and 9-143067, it is proposed that an external preparation effective for allergic dermatitis such as atopic dermatitis is implemented by blending DHA therein. The "Japanese Journal of Clinical Nutrition" (Vol. 84(2) 1994, P148), "Japanese Journal of Clinical and Experimental Medicine" (Vol.70(6) 1993, p263), and Japanese Patent Laid-Open Publication No. Hei 9-143067 disclose as follows. ArA which is an ω-6 highly unsaturated fatty acid plays an important role in the crisis of allergic inflammation. Further, ArA is converted to inflammation-inducing prostaglandin ($PGE_2$) or leukotriene ($LTB_4$, $LTC_4$, or $LTE_4$). The enzyme for carrying out the conversion (arachidonate cascade) is the same as the enzyme used for the conversion of EPA or DHA, i.e., ω-3 highly unsaturated fatty acid to the $PG_3$ strain or the $LT_5$ strain (not having an inflammation-inducing activity), and both act in an antagonistic manner to each other. Particularly, it is said that the ω-3 highly unsaturated fatty acid has a higher affinity than that of the ω-6 highly unsaturated fatty acid. Therefore, when both are allowed to coexist, the ω-3 highly unsaturated fatty acid will preferentially react.

On the other hand, it is known that the ω-3 highly unsaturated fatty acid such as ALA, EPA, or DHA shows an antiallergic effect even through oral administration with almost no side effect. As the reports on the treatment of allergy by using such highly unsaturated fatty acids, the following proposals have been made.

Japanese Patent Laid-Open Publication No. Sho 59-152324 discloses that an appropriate combination of the ω-3 highly unsaturated fatty acid and the ω-6 highly unsaturated fatty acid is effective for the treatment of allergic rhinitis, atopic dermatitis, or the like. The "Japanese Journal of Pediatric Allergy and Clinical Immunology" (Vol.6, pp87 - 91 (1992)), discloses that, a perilla frutescens oil rich in ALA is administered to a pediatric patient with atopic dermatitis, so that the improvement effect is obtained. The "Japanese Journal of Clinical Nutrition" (Vol. 87, pp185 - 189 (1995)), the "Japanese Journal of Pediatric Allergy and Clinical Immunology" (Vol.87, pp18 - 26 (1994), and the "Allergy in Practice" (Vol.14, pp296 - 305 (1994)) discloses that the improvement effect can be observed by administering an oil powder obtained from a perilla frutescens oil containing ALA and a refined fish oil containing EPA and DHA to a patient with atopic dermatitis. Japanese Patent Laid-Open Publication No. Hei 7-173060 discloses that atopic dermatitis can be treated by oral administration of a fish oil containing EPA and DHA.

Further, the factors affecting dermatitis in the lipid have been studied in the prior art. In general, it is said that a radical present in a lipoperoxide acts on the double bond in the lipid molecule, to consecutively cause hyperoxidation

by a chain reaction. The lipoperoxide irritates the skin, causing the aging and inflammation of the skin. For this reason, the lipoperoxide causes exasperation of the affected part, or causing a novel inflammation lesion. Therefore, for the lipid to be used for a skin external preparation, its peroxide value (below, abbreviated as POV) serving as an indicator of the lipoperoxide content of the oil and/or fat is desirably low.

As the indicator of the lipoperoxide content of the oil and fat, an aldehyde value (below, abbreviated as AlV) is also used other than the POV. The AlV is defined as the amount of aldehyde produced from further oxidation and decomposition of the lipoperoxide resulting from the oxidation of an oil and/or fat. Therefore, for the lipid to be used for a skin external preparation, its AlV serving as an indicator of the lipoperoxide content of the oil and/or fat is desirably low.

On the other hand, it is already known that a polymer containing 2-(meth)acryloyloxyethyl phosphorylcholine (below, abbreviated as MPC) as a structural unit is excellent as a skin external preparation such as a cosmetic material or a drug as shown below.

Japanese Patent Laid-Open Publication No. Hei 5-70321 discloses that a copolymer of MPC is excellent in hygroscopicity and moisture retention, and hence suitable for a cosmetic material excellent in moisture retention ability. Japanese Patent Laid-Open Publication Nos. Hei 6-157270 and 6-157271 disclose that a terpolymer of MPC, a hydrophobic monomer, and a hydrophilic monomer is used for a cosmetic material. Japanese Patent Laid-Open Publication No. Hei 6-178390 discloses a cosmetic material containing an MPC polymer and a liposome. Japanese Patent Laid-Open Publication No. Hei 7-1185123 discloses a cosmetic material in which an MPC polymer and a cosmetic powder are blended. Japanese Patent Laid-Open Publication No. Hei 9-52848 discloses a cosmetic material containing an MPC copolymer and mucopolysaccharide. Japanese Patent Laid-Open Publication No. Hei 6-157269 discloses that an MPC homopolymer is excellent in heat-retaining property, and improves the skin and hair conditions of skin roughening, insufficient gloss, and the like. Japanese Patent Laid-Open Publication No. Hei 9-315935 discloses that, by blending a polymer or copolymer of an MPC analog, and a water-soluble nitrogen-containing biopolymer in a cosmetic material for preventing skin irritation, an irritation-inhibiting effect derived from the MPC analog can be obtained. Japanese Patent Laid-Open Publication No. Hei 9-315949 discloses that, by blending a polymer or copolymer of an MPC analog in a cosmetic material for preventing skin irritation, it is possible to inhibit the irritation derived from a skin stimulus. Japanese Patent Laid-Open Publication No. Hei 10-45525 discloses that, by using an MPC homopolymer, a stable multilayer emulsified composition can be formed, and it is suitable for a cosmetic material or the like. Japanese Patent Laid-Open Publication No. Hei 10-45626 discloses that an MPC homopolymer shows a permeation promoting ability for a medicinal component, and hence it can be blended in a skin external base with high safety.

Incidentally, it is known that as an oxidation stabilizer for a fat or oil, particularly a fat or oil containing a highly unsaturated fatty acid, an antioxidant such as dibutylhydroxytoluene (below, abbreviated as BHT), butylhydroxyanisole (below, abbreviated as BHA), or tocopherol is used ("Yushi Kagaku Binran (Fat and Oil Chemistry Guidebook)" second edition, published by Yukagaku Kyokai Shuppan (Oil Chemists' Society), p61, p511 (1985).

As described above, it is known that the ω-3 unsaturated fatty acid, the ω-6 unsaturated fatty acid, the phosphorylcholine analog, or the antioxidant can each be blended in a cosmetic material. However, the cosmetic material in which a combination of the respective components is blended has not known specifically. Further, a cosmetic material in which a combination of three of a specific natural oil and/or fat having a high content of the ω-3 unsaturated fatty acid, a phosphorylcholine-like group containing polymer, and an antioxidant is blended have not been known, either. Further, the fact that more remarkable effects than ever can be obtained as a skin external preparation base when these three components are combined to form the skin external preparation base.

It is an object of the present invention to provide a skin external preparation composition with less side effects, effective for various dermatitises and the like, particularly effective for atopic dermatitis and the like.

It is another object of the present invention to provide a skin external preparation composition with less side effects, effective for various dermatitises and the like, particularly effective for atopic dermatitis and the like even if an adrenal cortical hormone and the like are not particularly blended therein.

The present invention provides a skin external preparation composition, comprising: a natural oil and/or fat (A) containing an ω-3 unsaturated fatty acid and an ω-6 unsaturated fatty acid, the content of the ω-3 unsaturated fatty acid being 4 times or more the content of the ω-6 unsaturated fatty acid; a phosphorylcholine-like group containing polymer (B), and an antioxidant (C).

Below, the present invention will be described in more details.

The skin external preparation composition of the present invention can be used as a base for a skin external preparation as a drug or a cosmetic material, which is expected to have preventing, inhibiting, or treating effects on, for example, skin roughening, crazing, capped skin, dermatitis, chilblain, eczema, itching, sore, miliaria, insect bite and sting, atopic dermatitis, or the like.

The skin external preparation composition of the present invention contains: a natural oil and/or fat (A) (below, may be abbreviated as "component A") containing an ω-3 unsaturated fatty acid and an ω-6 unsaturated fatty acid, the content of the ω-3 unsaturated fatty acid being 4 times or more the content of the ω-6 unsaturated fatty acid; a phosphorylcholine-like group containing polymer (B) (below, may be abbreviated as "component B"), and an antioxidant (C) (below, may be abbreviated as "component C") .

Examples of component A may include liver oil, tunny fish oil, perilla oil, perilla frutescens oil, linseed oil, or the like, in which the total content of the ω-3 unsaturated fatty acid is 4 times or more the total content of the ω-6 unsaturated fatty acid.

The liver oils are oils and/or fats extracted from the liver or pyloric appendage, such as a cod liver oil and a shark liver oil. These oils may be obtained by a known extraction method. The tunny fish oils usable may include fatty oils extracted from various tunny fishes. The tunny fish oils are desirably as fresh as possible. The perilla oil usable may include an oil obtained by subjecting a perilla plant including leaves, seeds, and the like to conventional steam distillation. Examples of the species of perilla may include Perilla frutescens Britton var crispa Decne, Perilla frutescence Crispa, and Perilla frutescens var. Britton kudo forma viridis Makino. The perilla frutescens oil usable may be a fatty oil obtained from the seed of perilla frutescens which is the stock seed of perilla. The linseed oil usable may be an oil and/or fat rich in unsaturated fatty acids obtained from the seed of flax.

In the component A to be used in the present invention, examples of the ω-3 unsaturated fatty acid may include ALA (18:3, ω-3), EPA (20:5, ω-3), docosapentaenoic acid (below, abbreviated as DPA) (22:5, ω-3), and DHA (22:6, ω-3), or mixtures thereof. Examples of the ω-6 unsaturated fatty acid may include linolic acid (18:2, ω-6). Further, the wording that the content of the ω-3 unsaturated fatty acid is 4 times or more the content of the ω-6 unsaturated fatty acid means as follows. That is, the content of the ω-3 unsaturated fatty acid is 4 times or more the content of the ω-6 unsaturated fatty acid based on the fatty acid composition of the raw material natural oil and/or fat on a weight basis.

The desirable fatty acid composition of the component A may contain DHA usually from 10 to 40 % by weight, and preferably from 15 to 30 % by weight; EPA usually from 5 to 20 % by weight, and preferably from 8 to 15 % by weight, and less than the amount of DHA; and ArA usually 5 % by weight or less, and preferably 1 % by weight or less. The content of DHA being less than 10 % by weight may result in poor treatment effect on skin diseases, whereas the content of DHA exceeding 40 % by weight may result in no further improvement of the treatment effect on skin diseases , both not being preferable. The content of EPA being less than. 5 % by weight may result in poor treatment effect on skin diseases, whereas the content of EPA exceeding 20 % by weight may result in no further improvement of the treatment effect on skin diseases, both not being preferable. If the content of ArA exceeds 5 % by weight, undesirably the treatment effects of DHA and EPA may be inhibited.

The lower the POV and AlV are, the more preferable they are. The more preferred component A is a natural oil and/or fat having the foregoing desirable fatty acid composition, and a POV of 30 meq/kg or less, and an AlV of generally 0.035 mmol/g or less, and preferably 0.030 mmol/g or less. If the AlV exceeds 0.035 mmol/g, undesirably, the smell derived from the lipoperoxide may occur, and the inflammation of skin may be promoted, particularly the hypersensitivity to ultraviolet rays may be increased due to the formation of the peroxides caused by ultraviolet rays arising from sunlight. Further, a liver oil having such physical property values is desirable.

Reductions in POV and AlV of the component A may be accomplished by the following method. That is, by using the liver of fish or the like as a raw material, the odor components of the peroxides and aldehyde compounds in the oil are removed by alkali hydrolysis, and separation and purification, and further by absorbent treatment and molecule distillation.

Since the component A is inferior in oxidation stability, preferably, it may be stored in a cool, dark place, or an antioxidant may be added thereto as early as possible.

In the skin external preparation composition of the present invention, the blending ratio of the component A is preferably from 90 to 99.999 % by weight based on the total amount of the components A and B. If the blending ratio of the component A is less than 90 % by weight, undesirably the improvement of the treatment effect on skin diseases may be insufficient. Further, the content of the ω-3 unsaturated fatty acid and the ω-6 unsaturated fatty acid of the component A is preferably from 40 to 100 % by weight. If it is less than 40 % by weight, undesirably, the desired effects may be insufficient.

The component B to be used in the present invention has no particular restriction as long as it is a polymer having a phosphorylcholine-like group. Examples thereof may include a polymer having the group represented by the formula (I) at its side chain (below, this polymer is abbreviated as a PC polymer):

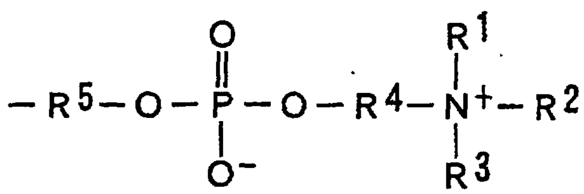 ...[I]

where $R^1$, $R^2$, and $R^3$ each denote an alkyl group having 1 to 8 carbon atoms; $R^4$ denotes $-(CH_2-CHR^6O)_m-(CH_2-CHR^6)-$ (where $R^6$ denotes a hydrogen atom, a methyl group or an ethyl group, and m denotes an integer of from 0 to 10.); and $R^5$ denotes $-(CH_2)_g-$ (where g is an integer of from 2 to 10).

The PC polymer may be obtained by, for example, subjecting a monomer having the group represented by the formula (I) to homopolymerization or copolymerization with other monomers. The monomer having the group represented by the formula (I) may have a polymerizable double bond and the group represented by the formula (I) in the molecule.

Examples of such a monomer may include 2-(meth)acrylolyloxyethyl-2'-(trimethylammonio)ethyl phosphate, 3-(meth)acrylolyloxypropyl-2'-(trimethylammonio)ethyl phosphate, 4-(meth)acrylolyloxybutyl-2'-(trimethylammonio) ethyl phosphate, 5-(meth) acrylolyloxypentyl-2'-(trimethylammonio)ethyl phosphate, 2-(meth)acrylolyloxyethyl-2'-(triethylammonio)ethyl phosphate, 3-(meth)acrylolyloxypropyl-2'-(triethylammonio)ethyl phosphate, 4-(meth)acrylolyloxybutyl-2'-(triethylammonio)ethyl phosphate, 5-(meth)acrylolyloxypentyl-2'-(triethylammonio)ethyl phosphate, 2-(meth)acrylolyloxyethyl-2'-(tripropylammonio)ethyl phosphate, 3-(meth)acrylolyloxypropyl-2'-(tripropylammonio)ethyl phosphate, 4-(meth)acrylolyloxybutyl-2'-(tripropylammonio)ethyl phosphate, 5-(meth)acrylolyloxypentyl-2'-(tripropylammonio)ethyl phosphate, 2-(meth)acrylolyloxyethyl-2'-(tributylammonio)ethyl phosphate, 3-(meth)acrylolyloxypropyl-2'-(tributylammonio)ethyl phosphate, 4-(meth)acrylolyloxybutyl-2'-(tripropylammonio)ethyl phosphate, 5-(meth) acrylolyloxypentyl-2'-(tributylammonio)ethyl phosphate, 2-(meth)acrylolyloxyethyl-3'-(trimethylammonio)propyl phosphate, 2-(meth)acrylolyloxyethyl-4'-(trimethylammonio)butyl ethyl phosphate, 2-(meth)acrylolyloxyethyl-3'-(triethylammonio)propyl phosphate, 2-(meth)acrylolyloxyethyl-4'-(triethylammonio) butyl phosphate, 2-(meth)acrylolyloxyethyl-3'-(tripropylammonio)propyl phosphate, 2-(meth)acrylolyloxyethyl-4'-(tripropylammonio)butyl phosphate, 2-(meth)acrylolyloxyethyl-3'-(tributylammonio)propyl phosphate, 2-(meth)acrylolyloxyethyl-4'-(tributylammonio)butyl phosphate, 3-(meth)acrylolyloxypropyl-3'-(trimethylammonio)propyl phosphate, 3-(meth)acrylolyloxypropyl-4'-(trimethylammonio) butyl phosphate, 3-(meth)acrylolyloxypropyl-3'-(triethylammonio)propyl phosphate, 3-(meth)acrylolyloxypropyl-4'-(triethylammonio)butyl phosphate, 3-(meth)acrylolyloxypropyl-3'-(tripropylammonio)propyl phosphate, 3-(meth)acrylolyloxypropyl-4'-(tripropylammonio)butyl phosphate, 3-(meth)acrylolyloxypropyl-3'-(tributylammonio)propyl phosphate, 3-(meth)acrylolyloxypropyl-4'-(tributylammonio)butyl phosphate, 4-(meth)acrylolyloxybutyl-3'-(trimethylammonio)propyl phosphate, 4-(meth)acrylolyloxybutyl-4'-(trimethylammonio)butyl phosphate, 4-(meth)acrylolyloxybutyl-3'-(triethylammonio)propyl ethyl phosphate, 4-(meth)acrylolyloxybutyl-4'-(triethylammonio)butyl phosphate, 4-(meth)acrylolyloxybutyl-3'-(tripropylammonio)propyl phosphate, 4-(meth)acrylolyloxybutyl-4'-(tripropylammonio)butyl phosphate, 4-(meth)acrylolyloxybutyl-3'-(tributylammonio)propyl phosphate, and 4-(meth)acrylolyloxybutyl-4'-(tributylammonio) butyl phosphate. The examples of the monomer may further include derivatives of maleic acid, fumaric acid, and itaconic acid with one to two groups represented by the formula (I) esterified thereto.

In manufacturing of the PC polymer, one or more of these monomers may be used.

The monomer having the group represented by the formula (I) is preferably the monomer represented by the formula (II) in terms of availability:

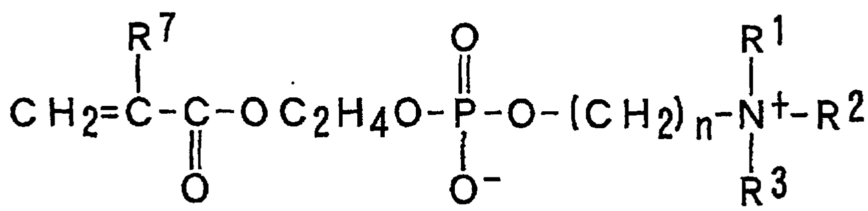

···[II]

where, $R^1$, $R^2$, and $R^3$ are the same or different groups, and each denote an alkyl group having 1 to 8 carbon atoms, and n denotes an integer of from 2 to 4; and $R^7$ denotes a hydrogen atom or a methyl group. Out of these, mention may be preferably made of 2-methacryloyloxyethyl-2'-(triethylammonio)ethyl phosphate (below, abbreviated as MPC) wherein $R^1 = R^2 = R^3$ is a methyl group, $R^7$ is a methyl group, and n is 2 in the formula (II).

The PC polymer is preferably a polymer produced by radical-polymerizing a monomer mixture containing 100 to 300 % by mole of a monomer having the group represented by the formula (I), and 0 to 70 % by mole of other monomers.

Examples of the other monomers may include alkyl (meth)acrylates such as methyl (meth)acrylate, ethyl (meth)acrylate, n-butyl(meth)acrylate, 2-ethylhexyl (meth)acrylate, n-dodecyl (meth)acrylate, and cyclohexyl (meth) acrylate; silyl group-containing (meth)acrylates such as 3-((meth)acryloyloxypropyl) trimethoxysilane, 3-((meth)acryloyloxypropyl) triethoxysilane, and 3-((meth)acryloyloxypropyl) tripropyloxysilane; fluorine-containing (meth)acrylates such as 2-(perfluorohexyl)ethyl (meth)acrylate, 2-(perfluorooctyl)ethyl (meth)acrylate, 1H, 1H, 5H-octafluoropentyl

(meth)acrylate, 1H, 1H, 7H-dodecafluoroheptyl (meth)acrylate, and 2,2,2-trifluoro-1-trifluoromethylethyl (meth)acrylate; hydroxyl group-containing (meth)acrylates such as 2-hydroxyethyl (meth)acrylate, 2-hydroxypropyl (meth)acrylate, polyethylene glycol mono(meth)acrylate, and polypropylene glycol mono(meth)acrylate; amide-containing monomers such as (meth)acrylic amide and N,N-dimethyl (meth)acrylic 5 amide; and (meth)acrylic acids.

Examples of the other monomers may further include substituted or unsubstituted styrene monomers such as styrene, methyl styrene, and chloromethyl styrene; vinyl ether monomers such as ethyl vinyl ether, and butyl vinyl ether; vinyl ester monomers such as vinyl acetate; vinyl silane monomers such as trimethoxy vinyl silane and triethoxy vinyl silane; substituted or unsubstituted hydrocarbon monomers such as ethylene, propylene, isobutylene, vinyl chloride, and vinylidene chloride; dibasic acid ester monomers such as diethyl fumarate and diethyl maleate; and N-vinyl pyrrolidone.

Preferred examples of the other monomers may still further include hydroxyl group-containing (meth)acrylates, alkyl (meth)acrylates, styrene monomers, and vinyl silane monomers. More preferred are methacrylic acid esters having alkyl groups or hydroxyalkyl groups having 1 to 8 carbon atoms from the viewpoint of characteristics.

The weight average molecular weight of the component B is desirably from 5000 to 5000000. The weight average molecular weight thereof may be variously adjusted according to the uses, but it may usually be from 10000 to 1000000, and preferably from 50000 to 500000 in terms of polyethylene glycol when the feeling aspect, gelling ability, and the like are considered.

The molar ratio of the structural units based on the phosphorylcholine-like group containing monomers to the structural units based on other monomers such as hydrophobic (meth)acrylic acid esters is preferably from 30 : 70 to 100 : 0. More preferably, it is from 50 : 50 to 97 : 3. If the molar ratio of the structural units based on the phosphorylcholine-like group containing monomers is less than 30, undesirably, the moisture retention effect and keeping of a beautiful skin based on the component B may be reduced.

The blending ratio of the component B is preferably from 10 to 0.001 % by weight based on the total amount of the components A and B. If it is beyond this range, undesirably, the skin beautifying effects such as improving roughening and insufficient gloss, and ensuring the retention of moisture and preventing the transpiration may be reduced.

Examples of the component C include vitamin C, dibutylhydroxytoluene (below, abbreviated as BHT, and also referred to as 2,6-di-t-butyl-p-cresol, or butylhydroxytoluene), butylhydroxyanisole (below, abbreviated as BHA), and citric acid. Particularly, BHT is preferably used.

The blending ratio of the component C is preferably from 0.001 to 1 part by weight, and preferably from 0.01 to 0.1 part by weight based on 100 parts by weight of the total amount of the components A and B. If it is less than 0.001 part by weight, the anti-oxidant effect on the component A may be insufficient, whereas, blending ratio exceeding 1 part by weight may result in no further improvement of the anti-oxidant effect.

The blending ratio of the composition of the present invention in a skin external preparation as a base thereof has no particular restriction, and it can be appropriately selected according to the form, effect, and the like of the skin external preparation.

The skin external preparation composition of the present invention may be formed in an appropriate formulation with other skin external base. It can be formed, for example, in liquid, gel, paste, cream, powder, or solid form, or the like. Further, when it is used for a skin external drug or skin external cosmetic material, the composition of the present invention can be appropriately mixed with components of commonly used other external bases, surfactants, fats and oils, moisture-retaining agents, stabilizers, antiseptics, thickeners, coloring matters and perfumes, refrigerants, and the like.

Examples of the other external bases may include white petrolatum, cetanol, liquid paraffin, squalene, squalane, lanoline, and medium chain fatty acid triglyceride.

Examples of the surfactants may include glycerin fatty acid ester, sucrose fatty acid ester, sorbitan fatty acid ester, lecithin, polyoxyethylene (below, abbreviated as POE) hardened castor oil, and sodium laurate.

Examples of the surfactants may include tetra-, or di-alkali salts of ethylenediaminetetraacetic acid (below, abbreviated as EDTA), and POE sorbitan fatty acid esters.

Examples of the antiseptics may include paraoxybenzoic acid ester, sorbic acid, phenol, and benzyl alcohol.

Examples of the perfumes and refrigerants may include peppermint oil, eucalyptus oil, menthol, rose oil, lavender oil, orange oil, vanilla flavor, fruit flavor, and vanillin.

Examples of the thickeners may include gum arabic, guar gum, carageenan, carboxymethyl cellulose, vinyl acetate resin emulsion, and polyacrylic acid salts.

Further, various medicinal components may also be blended therein. For example, bufexamac, allantoin, dipotassium glycyrrhizate, lidocaine, or the like, having an antipruritic action may also be blended therein.

The skin external preparation of the present invention may be blended in cosmetic materials for skin care such as ointments, lotions, milk lotions, foundation, lip rouge, moisture-retaining cream, cold cream, hand cream, and massage materials; for body care such as cosmetic bases and patch preparations; for hair care such as lotion and

shampoo; for eye care such as eyeliner, mascara, and eye shadow; and for make-up such as cheek rouge, nail polish, and face powder.

Below, as one example thereof, a description will be given to the formulation and the manufacturing method of an ointment in which the composition of the present invention is blended.

For example, white petrolatum, stearyl alcohol, and polyoxyethylene (60 mol adduct) hardened castor oil are taken in a vessel, heated to 70 °C, and homogenized with stirring. The component A to which the component C such as BHT and BHA have previously been added is added thereto. On the other hand, propylene glycol is heated to 70 °C, and an antiseptic such as methyl paraoxybenzoate or propyl paraoxybenzoate is added thereto and mixed therewith. The component B and purified water are added thereto, and mixed to be an emulsion by means of a homomixer. When a water-soluble component C such as vitamin C or citric acid is used, the component C may be previously dissolved in purified water to be used. The resulting emulsion and the mixture containing the components A and C previously prepared are emulsified with stirring by means of a paddle mixer. Then, the resulting emulsion is cooled, and well mixed until it is hardened. In the foregoing manner, an ointment may be manufactured.

As the method of using the ointment, for example, the conventional using method is as follows. That is, the ointment is spread so as to cover the whole affected part of a patient, and lightly rubbed therein if possible depending on the symptoms. When it is impossible to lightly rub it therein, the ointment is preferably used in the following manner. That is, a gauze or the like to which a required amount of the ointment depending on the symptom of a patient has been applied is fixed on the affected part with a tape at a rate of about 1 to 3 times per day.

Since the skin external preparation composition of the present invention contains the components A to C, the inhibition and treatment effects thereof on the skin inflammation are remarkable. Further, the composition can soothe itching due to inflammation, and reduce the influences by ultraviolet rays. Still further, it ensures the stabilization of the product over a long period of time, and it can inhibit the occurrence of the odor components derived from lipoperoxide generated by oxidation.

Below, the present invention will be described in more details by way of the following examples. However, the present invention is not limited thereto.

The measurements of POV, fatty acid composition, and AIV were carried out in the following manner.

1. Measurement of POV

The measurement of POV was carried out in the following manner in accordance with the method for standard oil and fat assay (2.5.2.1-1996) prescribed by the Japan Oil Chemists's Society.

A sample is taken in an Erienmeyer flask equipped with a ground stopper, and 50 ml of an acetic acid - isooctane (3:2, V/V) mixed solvent is added thereto with gently stirring so as to completely dissolve the sample therein. The air in the Erlenmeyer flask is replaced with a nitrogen gas. Further, 0.1 ml of a saturated potassium iodide solution is added thereto with passing a nitrogen gas therethrough. The flask is stoppered and stirred, and then 30 ml of water is added thereto with vigorously stirring for 10 seconds. The resulting solution is titrated with a 0.01 ml/l sodium thiosulfate standard solution. When the solution shows a slightly tinted yellow color, 0.5 ml of a starch solution is added thereto to continue the titration, and the instant when the blue color of the measuring solution has disappeared is taken as the end point. POV is calculated by the following formula. POV(meq/kg) = Ap X F X 10/Sp

Ap: Amount of 0.01 ml/l sodium thiosulfate standard solution (ml), Sp: Amount of Sample taken (g), F: Factor of sodium thiosulfate standard solution.

2. Method for measuring the fatty acid composition (DHA, EPA, ArA, ALA, linolic acid, and the like)

About 500 mg of a natural oil and/or fat is taken in a 50-ml eggplant-shape flask equipped with a ground stopper and a cooling tube for esterification, and 6 ml of a 1/2 N sodium hydroxide-methanol solution is added thereto. The mixture is heated over a water bath for about 7 minutes until oil droplets disappear to prepare a uniform solution. Then, 7 ml of a boron trifluoride - methanol reagent is added thereto, and the mixture is boiled for 2 minutes. Thereafter, 5 ml of hexane is added from the top of the cooling tube, and the mixture is boiled for another 1 minute. After finishing heating, purified water is added thereto until the hexane solution reaches the neck of the flask, and the hexane solution is collected. The hexane solution is admixed with about 10 ml of purified water with stirring, and then allowed to stand for layer separation. After removing the water layer, the same operation is repeated three times. After the washing operation, sodium sulfate is added to the hexane solution for dehydration, and then sodium sulfate is filtered off, to obtain a sample for gas chromatography. The sample is analyzed by a gas chromatographer (5890 manufactured by Hewlett-packard Company) equipped with a DB-WAX capillary column (column manufactured by J & W Scientific Co., (internal diameter: 0.25 mm, length: 30 m), with 0.25mm-thick polyethylene glycol as a solid phase liquid). Temperature of the inlet and the detector is set at 250 °C, and the column temperature is heated at 5 °C /minute up to from 140 °C to 210 °C. A flame ionization detector is used for detection and a helium gas is used as a carrier gas. For respective

fatty acids of DHA, EPA, ArA, ALA, and linolic acid, each content of the fatty acids (% by weight) is calculated from the peak area percent of the methyl esterified product thereof.

3. Measurement of AlV

(Preparation of preliminary sample solution)

One gram of a sample is taken and admixed with ethyl acetate to bring the total amount to 10 ml with precision.

(Preparation of reaction sample solution)

40 mg of 4-amino-3-penten-2-one (Fluoral-P) is taken and admixed with ethyl acetate to bring the total amount to 10 ml with precision.

(Preparation of preliminary standard sample solution)

0.1 g of a standard reference material, 2-hexenal is taken and admixed with ethyl acetate to bring the total amount to 10 ml with precision.

(Method)

One ml of a preliminary sample solution, 1 ml of a reaction sample solution, and 5 ml of acetic acid are taken in a 10-ml test tube equipped with a ground stopper, to prepare a sample solution. Nitrogen is bubbled into the solution at a flow rate of 10 ml/min for 1 minute, and then the air gap above the solution is replaced by nitrogen at a flow rate of 30 ml/min for 1 minute, and a cap is put thereon to air-tightly stopper the test tube. The solution in the test tube is allowed to react in a 60 °C water bath for 1 hour. After the completion of the reaction, the test tube is immersed in an ice bath to stop the reaction, and the solution is returned to room temperature immediately before the measurement. The sample solution returned to room temperature is placed in a quarts cell of 1 cm X 1 cm in layer length, and the fluorescence strength of the sample solution ($F_T$) is determined at an excitation wavelength of 410 nm and a fluorescence wavelength of 500 nm with a fluorescence spectrophotometer.

Separately, 1 ml of a preliminary standard solution, 1 ml of a reaction sample solution, and 5 ml of acetic acid are taken in a 10-ml test tube equipped with a ground stopper, to prepare a standard solution. The measurement is carried out in the same manner to determine the fluorescence strength of the standard solution (Fs).

For comparison, a fluorescence blank solution composed of 1 ml of a reaction sample solution, 1 ml of ethyl acetate and 5 ml of acetic acid, a sample blank solution composed of 1 ml of a preliminary sample solution, 1 ml of ethyl acetate and 5 ml of acetic acid, and a standard reference material blank solution composed of 1 ml of preliminary standard solution, 1 ml of ethyl acetate and 5 ml of acetic acid are prepared respectively. The same operation is carried out for each solution to determine the fluorescence strengths of the fluorescence blank solution, the sample blank solution, and the standard reference material blank solution ($F_B$, $F_{TB}$, and $F_{SB}$). From the measurement results, AlV is calculated according to the following calculation equation:

$$AlV(mmol/g)=((F_T-F_{TB}-F_B)/(F_S-F_{SB}-F_B)) \times ((W_S)/(W_T)) \times 10 \times 0.1018$$

$F_T$: fluorescence strength (500 nm) of sample solution, $F_S$: fluorescence strength (500 nm) of standard solution, $F_B$: fluorescence strength (500 nm) of fluorescence blank solution, $F_{TB}$: fluorescence strength (500 nm) of sample blank solution, $F_{SB}$: fluorescence strength (500 nm) of standard reference material blank solution, $W_S$: amount (g) of standard reference material taken, and $W_T$: amount (g) of sample taken.

Synthesis Example 1; (MPC/BMA = 50/50 polymer) : (synthesis of PC-1)

First, 14.85 g (50 mmol) of MPC and 7.1 g (50 mmol) of N-butyl methacrylate (BMA) were weighed in a polymerization tube made of glass, and 0.82 g of azobisisobutylonitrile was added thereto as a polymerization initiator. The resulting mixture was dissolved in 100 ml of ethanol. An argon gas was blown into the solution for 5 minutes, and the polymerization tube was then heat-sealed, and the polymerization reaction was performed at 60 °C for 6 hours. After the completion of the reaction, the reaction mixture was added dropwise to an ether. The precipitated copolymer was filtered off, so that the unreacted monomer was removed, followed by drying under reduced pressure, to obtain

an MPC-BMA copolymer (below, the resulting polymer is referred to as PC-1). The yield of the PC-1 and the MPC composition ratio were determined by phosphorus fixation. Further, the weight-average molecular weight was determined with standard polyethylene glycol by gel permeation chromatography (GPC). The analysis results are shown in Table 1.

Synthesis Example 2; (MPC/BMA = 80/20 polymer) : (synthesis of PC-2)

The reaction was performed in the same manner as in Synthesis Example 1, except that the amount of MPC used and the amount of BMA used in Synthesis Example 1 were changed into 23.80 g (80 mmol) and 2.82 g (20 mmol), respectively, and further, the reaction time and the amount of the polymerization initiator were adjusted, to obtain an MPC-BMA copolymer (below, the resulting polymer is referred to as PC-2). The same measurements as in Synthesis Example 1 were carried out for the PC-2. The results are shown in Table 1.

Synthesis Example 3; (MPC/BMA = 100/0 polymer) : (synthesis of PC-3)

The reaction was performed in the same manner as in Synthesis Example 1, except that the composition shown in Table 1 was adopted, and further, the reaction time and the amount of the polymerization initiator were adjusted, to obtain an MPC homopolymer (below, the resulting polymer is referred to as PC-3). Various measurements were carried out therefor. The results are shown in Table 1.

Synthesis Examples 4 to 7; (MPC/EA, MPC/EHA, MPC/St polymer) : (synthesis of PCs-4 to 7)

The polymerization was respectively carried out in the same manner as in Synthesis Example 1, except that ethyl acrylate (below, abbreviated as EA), 2-ethylhexyl acrylate (below, abbreviated as EHA), or styrene (below, abbreviated as St) was used in such an amount as shown in Table 1 in place of BMA used in Synthesis Example 1. Consequently, polymers were obtained, and various measurements were carried out therefor. The results are shown in Table 1. The abbreviation of the polymer obtained in each synthesis example is shown in Table 1.

Table 1

| | Synthesis Example | | | | | | |
|---|---|---|---|---|---|---|---|
| | 1 | 2 | 3 | 4 | 5 | 6 7 | |
| Abbreviation of polymer | PC-1 | PC-2 | PC-3 | PC-4 | PC-5 | PC-6 | PC-7 |
| Component a:MPC(g) | 14.85 | 23.80 | 29.70 | 28.81 | 20.79 | 5.94 | 8.91 |
| Component b:<br>Comonomer(g)<br>BMA<br>EA<br>EHA<br>St | <br><br>7.1<br>-<br>-<br>- | <br><br>2.82<br>-<br>-<br>- | <br><br>-<br>-<br>-<br>- | <br><br>-<br>0.30<br>-<br>- | <br><br>-<br>-<br>5.55<br>- | <br><br>-<br>-<br>-<br>8.32 | <br><br>-<br>7.07<br>-<br>- |
| Charge molar ratio (a/b) | 50/50 | 80/20 | 100/0 | 97/3 | 70/30 | 40/60 | 30/70 |
| Yield (%) | 65 | 68 | 70 | 64 | 63 | 69 | 70 |
| Polymer molar ratio (a/b) | 49/51 | 79/21 | 100/0 | 96.8/ 3.2 | 69.2/ 30.8 | 39.8/ 60.2 | 31/69 |
| Weight-average molecular weight | 146000 | 153000 | 47000 | 150000 | 550000 | 25000 | 40000 |

Example 1; ointment formulation 1

One hundred grams of liquid paraffin, 100 g of cetanol, 50 g of isopropyl myristate, 30 g of oleyl alcohol, 10 g of POE (40) glycol stearate, 10 g of sucrose fatty acid ester, 1 g of methyl paraoxybenzoate, and 1 g of propyl paraoxybenzoate were weighed and placed in a vessel, and heated to 70 °C to be molten for preparing an ointment base. Then, a mixture of 0.1 mg of BHT as the component C, 95 g of a liver oil as the component A shown in Table 2, and 2 g of a flavoring agent (peppermint oil) was added thereto.
Then, to the resulting mixture, was added the product which had been previously obtained by mixing 546 g of purified water, 50 g of concentrated glycerin, and 5 g of the PC-1 prepared as the component B in Synthesis Example

1 and had been heated to 70 °C. The mixture was gradually cooled with stirring in a paddle mixer at a rate of 50 rpm to manufacture an ointment. The resulting ointment was charged respectively in 30g vials, resulting in samples. The formulation is shown in Table 2.

Examples 2 to 18, and Comparative Examples 1 to 7

Ointments were manufactured in the same manner as in Example 1 in accordance with their respective formulations shown in Tables 2 to 5. The formulations are shown in Tables 2 to 5.

Table 2

| | | Examples | | | | | |
|---|---|---|---|---|---|---|---|
| | | 1 | 2 | 3 | 4 | 5 | 6 |
| Component A: Natural oil or fat | | Liver oil | Tunny fish oil | Tunny fish oil | Peri-lla oil | P. frutescens oil | Lin-seed oil |
| Blending amount (g) | | 95 | 95 | 99 | 99 | 90 | 99.9 |
| Natural oil and fat Properties | α-Linolenic acid (ω-3) | 1 | 2 | 2 | 62 | 66 | 54 |
| | Linoleic acid (ω-6) | 2 | 2 | 2 | 13 | 16 | 12 |
| | DHA(ω-3) | 28 | 30 | 30 | 0 | 0 | 0 |
| | EPA(ω-3) | 15 | 18 | 18 | 0 | 0 | 0 |
| | ArA(ω-6) | 1 | 2 | 2 | 0 | 0 | 0 |
| | POV(meq/kg) | 2 | 1 | 1 | 0.5 | 0.5 | 0.5 |
| | AlV(mmol/g) | 0.025 | 0.02 | 0.02 | 0.01 | 0.01 | 0.01 |
| | ω-3FA/ω-6FA ratio | 44/3= 14.7 | 50/4= 12.5 | 50/4= 12.5 | 62/13 =4.77 | 66/16 =4.13 | 54/12 =4.5 |
| Component B: type | | PC-1 | PC-1 | PC-1 | PC-1 | PC-1 | PC-1 |
| Blending amount (g) | | 5 | 5 | 1 | 1 | 10 | 0.1 |
| Weight % of component A/(A+B) | | 95 | 95 | 99 | 99 | 90 | 99.9 |
| Weight % of component B/(A+B) | | 5 | 5 | 1 | 1 | 10 | 0.1 |
| Component C: antioxidant type | | BHT | BHT | BHT | BHT | BHT | BHT |
| Blending amount (g) | | 0.1 | 0.1 | 0.1 | 0.1 | 0.1 | 0.1 |
| Weight % of component C/(A+B) | | 0.001 | 0.001 | 0.001 | 0.001 | 0.001 | 0.001 |
| Ointment base weight (g) | | 354 | 354 | 354 | 354 | 354 | 354 |

Table 3

| | | Examples | | | | | |
|---|---|---|---|---|---|---|---|
| | | 7 | 8 | 9 | 10 | 11 | 12 |
| Component A: Natural oil or fat | | Liver oil | Tunny fish oil | Tunny fish oil | Peri-lla oil | P. frute scens oil | Lin-seed oil |
| Blending amount (g) | | 95 | 95 | 99 | 99 | 90 | 99.9 |
| Natural oil and fat Properties | α-Linolenic acid (ω-3) | 1 | 2 | 2 | 62 | 66 | 54 |
| | Linoleic acid (ω-6) | 2 | 2 | 2 | 13 | 16 | 12 |
| | DHA(ω-3) | 28 | 30 | 30 | 0 | 0 | 0 |
| | EPA(ω-3) | 15 | 18 | 18 | 0 | 0 | 0 |
| | ArA(ω-6) | 1 | 2 | 2 | 0 | 0 | 0 |
| | POV(meq/kg) | 2 | 1 | 1 | 0.5 | 0.5 | 0.5 |
| | AlV(mmol/g) | 0.025 | 0.02 | 0.02 | 0.01 | 0.01 | 0.01 |
| | ω-3FA/ω-6FA ratio | 44/3= 14.7 | 50/4= 12.5 | 50/4= 12.5 | 62/13 =4.77 | 66/16 =4.13 | 54/12 =4.5 |
| Component B: type | | PC-2 | PC-2 | PC-2 | PC-2 | PC-2 | PC-2 |
| Blending amount (g) | | 5 | 5 | 1 | 1 | 10 | 0.1 |
| Weight % of component A/(A+B) | | 95 | 95 | 99 | 99 | 90 | 99.9 |
| Weight % of component B/(A+B) | | 5 | 5 | 1 | 1 | 10 | 0.1 |
| Component C: antioxidant type | | BHT | BHT | BHT | BHT | BHT | BHT |
| Blending amount (g) | | 0.1 | 0.1 | 0.1 | 0.1 | 0.1 | 0.1 |
| Weight % of component C/(A+B) | | 0.001 | 0.001 | 0.001 | 0.001 | 0.001 | 0.001 |
| Ointment base weight (g) | | 354 | 354 | 354 | 354 | 354 | 354 |

Table 4

| | | Examples | | | | | |
|---|---|---|---|---|---|---|---|
| | | 13 | 14 | 15 | 16 | 17 | 18 |
| Component A: Natural oil or fat | | Liver oil | Tunny fish oil | Tunny fish oil | Peri-lla oil | P. frute scens oil | Lin-seed oil |
| Blending amount (g) | | 99.975 | 99.98 | 99.98 | 99.99 | 99.99 | 99.99 |
| Natural oil and fat Properties | α-Linolenic acid (ω-3) | 1 | 2 | 2 | 62 | 66 | 54 |
| | Linoleic acid (ω-6) | 2 | 2 | 2 | 13 | 16 | 12 |
| | DHA(ω-3) | 28 | 30 | 30 | 0 | 0 | 0 |
| | EPA(ω-3) | 15 | 18 | 18 | 0 | 0 | 0 |
| | ArA(ω-6) | 1 | 2 | 2 | 0 | 0 | 0 |
| | Oleic acid | 19 | 19 | 17 | 85 | 7 | 38 |
| | γ-Lino-lenic acid | 0 | 0 | 0 | 0 | 10 | 0 |
| | POV(meq/kg) | 2 | 1 | 1 | 0.5 | 0.5 | 0.5 |
| | AlV(mmol/g) | 0.025 | 0.02 | 0.02 | 0.01 | 0.01 | 0.01 |
| | ω-3FA/ω-6FA ratio | 44/3= 14.7 | 50/4= 12.5 | 50/4= 12.5 | 62/13 =4.77 | 66/16 =4.13 | 54/12 =4.5 |
| Component B: type | | PC-3 | PC-3 | PC-3 | PC-3 | PC-3 | PC-3 |
| Blending amount (g) | | 0.025 | 0.02 | 0.02 | 0.01 | 0.01 | 0.01 |
| Weight % of component A/(A+B) | | 99.97 | 99.98 | 99.98 | 99.99 | 99.99 | 99.99 |
| Weight % of component B/(A+B) | | 0.03 | 0.02 | 0.02 | 0.01 | 0.01 | 0.01 |
| Component C: antioxidant type | | BHT | BHT | BHT | BHT | BHT | BHT |
| Blending amount (g) | | 0.1 | 0.1 | 0.1 | 0.1 | 0.1 | 0.1 |
| Weight % of component C/(A+B) | | 0.001 | 0.001 | 0.001 | 0.001 | 0.001 | 0.001 |
| Ointment base weight (g) | | 354 | 354 | 354 | 354 | 354 | 354 |

Table 5

| | | Comparative Examples | | | | | | |
|---|---|---|---|---|---|---|---|---|
| | | 1 | 2 | 3 | 4 | 5 | 6 | 7 |
| Component A: Natural oil or fat | | Liver oil | Liver oil | Sardine oil | Olive oil | Evening primrose oil | Corn oil | Soybean oil |
| Blending amount (g) | | 100 | 95 | 95 | 95 | 95 | 95 | 95 |
| Natural oil and fat Properties | α-Linolenic acid (ω-3) | 1 | 1 | 1 | 0 | 1 | 0 | 13 |
| | Linoleic acid (ω-6) | 2 | 2 | 4 | 7 | 73 | 51 | 62 |
| | DHA(ω-3) | 28 | 28 | 9 | 0 | 0 | 0 | 0 |
| | EPA(ω-3) | 15 | 15 | 13 | 0 | 0 | 0 | 0 |
| | ArA(ω-6) | 1 | 1 | 2 | 0 | 0 | 0 | 0 |
| | Oleic acid | 19 | 19 | 17 | 85 | 7 | 38 | 13 |
| | γ-Linolenic acid | 0 | 0 | 0 | 0 | 10 | 0 | 0 |
| | POV(meq/kg) | 2 | 35 | 1 | 0.5 | 0.5 | 0.3 | 0.5 |
| | AlV(mmol/g) | 0.025 | 0.13 | 0.02 | 0.01 | 0.01 | 0.01 | 0.01 |
| | ω-3FA/ω-6FA ratio | 44/3 = 14.7 | 44/3 = 14.7 | 23/6 =3.8 | 0/7 =0 | 1/73 = 0.01 | 0/51 =0 | 13/62 = 0.21 |
| Component B: type | | - | PC-1 | PC-1 | PC-1 | PC-1 | PC-1 | PC-1 |
| Blending amount (g) | | | 5 | 5 | 5 | 5 | 5 | 5 |
| Weight % of component A/(A+B) | | 100 | 95 | 95 | 95 | 95 | 95 | 95 |
| Weight % of component B/(A+B) | | 0 | 5 | 5 | 5 | 5 | 5 | 5 |
| Component C: antioxidant type | | BHT | - | BHT | BHT | BHT | BHT | α-Tocopherol |
| Blending amount (g) | | 0.1 | | 0.1 | 0.1 | 0.1 | 0.1 | 0.1 |
| Weight % of component C/(A+B) | | 0.001 | 0 | 0.001 | 0.001 | 0.001 | 0.001 | 0.001 |
| Ointment base weight (g) | | 354 | 354 | 354 | 354 | 354 | 354 | 354 |

In Tables 2 to 5, natural oil and fat properties other than POV and AlV are expressed in purity measured by gas chromatography (% by weight).

Test Examples 1 to 22

First, 220 subjects having skin diseases such as skin roughening and psoriasis disease were divided into 22 groups (10 subjects per group) as a panel. Then, tests using the ointments prepared in Examples 2 to 6, and Examples 8 to 18, and Comparative Examples 1 to 7 were carried out therefor. The test method is as follows.

(Test method 1)

The ointments were applied to the affected parts of the subjects two times per day for eight consecutive weeks. The degree of improvement of skin roughening or psoriasis disease before and after use was observed by visual inspection after 4 weeks and after 8 weeks for evaluation. The results are shown in Tables 6 to 13. In Tables, the number of subjects improved after 8 weeks includes the number of subjects improved after 4 weeks.

Table 6

| Skin roughening improvement test | | Test Examples | | | | |
|---|---|---|---|---|---|---|
| | | 1 | 2 | 3 | 4 | 5 |
| Ointment used | | Example 2 | Example 3 | Example 4 | Example 5 | Example 6 |
| Evaluation (No.of persons) | Improvement after 4 weeks | 7 | 8 | 8 | 6 | 8 |
| | Improvement after 8 weeks | 8 | 9 | 9 | 8 | 8 |
| | No change | 2 | 1 | 1 | 2 | 2 |
| | Dis-continuation of treatment due to exacerbation | 0 | 0 | 0 | 0 | 0 |
| | Total | 10 | 10 | 10 | 10 | 10 |

Table 7

| Psoriasis disease improvement test | | Test Examples | | | | |
|---|---|---|---|---|---|---|
| | | 1 | 2 | 3 | 4 | 5 |
| Ointment used | | Example 2 | Example 3 | Example 4 | Example 5 | Example 6 |
| Evaluation (No.of persons) | Improvement after 4 weeks | 7 | 8 | 6 | 7 | 6 |
| | Improvement after 8 weeks | 9 | 9 | 9 | 8 | 9 |
| | No change | 1 | 1 | 1 | 2 | 1 |
| | Dis-continuation of treatment due to exacerbation | 0 | 0 | 0 | 0 | 0 |
| | Total | 10 | 10 | 10 | 10 | 10 |

Table 8

| Skin roughening improvement test | | Test Examples | | | | |
|---|---|---|---|---|---|---|
| | | 6 | 7 | 8 | 9 | 10 |
| Ointment used | | Example 8 | Example 9 | Example 10 | Example 11 | Example 12 |
| Evaluation (No.of persons) | Improvement after 4 weeks | 6 | 7 | 6 | 5 | 8 |
| | Improvement after 8 weeks | 9 | 8 | 9 | 8 | 9 |
| | No change | 1 | 2 | 1 | 2 | 1 |
| | Dis-continuation of treatment due to exacerbation | 0 | 0 | 0 | 0 | 0 |
| | Total | 10 | 10 | 10 | 10 | 10 |

Table 9

| Psoriasis disease improvement test | | Test Examples 6 | 7 | 8 | 9 | 10 |
|---|---|---|---|---|---|---|
| Ointment used | | Example 8 | Example 9 | Example 10 | Example 11 | Example 12 |
| Evaluation (No.of persons) | Improvement after 4 weeks | 7 | 7 | 7 | 5 | 6 |
| | Improvement after 8 weeks | 8 | 9 | 8 | 9 | 10 |
| | No change | 2 | 1 | 2 | 1 | 0 |
| | Dis-continuation of treatment due to exacerbation | 0 | 0 | 0 | 0 | 0 |
| | Total | 10 | 10 | 10 | 10 | 10 |

Table 10

| Skin roughening improvement test | | Test Examples 11 | 12 | 13 | 14 | 15 |
|---|---|---|---|---|---|---|
| Ointment used | | Example 13 | Example 14 | Example 15 | Example 16 | Example 17 |
| Evaluation (No.of persons) | Improvement after 4 weeks | 6 | 8 | 7 | 5 | 7 |
| | Improvement after 8 weeks | 9 | 10 | 9 | 9 | 9 |
| | No change | 1 | 0 | 1 | 1 | 1 |
| | Dis-continuation of treatment due to exacerbation | 0 | 0 | 0 | 0 | 0 |
| | Total | 10 | 10 | 10 | 10 | 10 |

Table 11

| Psoriasis disease improvement test | | Test Examples | | | | |
|---|---|---|---|---|---|---|
| | | 11 | 12 | 13 | 14 | 15 |
| Ointment used | | Example 13 | Example 14 | Example 15 | Example 16 | Example 17 |
| Evaluation (No.of persons) | Improvement after 4 weeks | 8 | 8 | 5 | 7 | 7 |
| | Improvement after 8 weeks | 9 | 8 | 9 | 9 | 10 |
| | No change | 1 | 2 | 1 | 1 | 0 |
| | Dis-continuation of treatment due to exacerbation | 0 | 0 | 0 | 0 | 0 |
| | Total | 10 | 10 | 10 | 10 | 10 |

Table 12

| Skin roughening improvement test | | Test Examples | | | | | | |
|---|---|---|---|---|---|---|---|---|
| | | 16 | 17 | 18 | 19 | 20 | 21 | 22 |
| Ointment used | | Comp. Ex.1 | Comp. Ex.2 | Comp. Ex.3 | Comp. Ex.4 | Comp. Ex.5 | Comp. Ex.6 | Comp. Ex.7 |
| Evaluation (No.of persons) | Improvement after 4 weeks | 1 | 0 | 2 | 2 | 1 | 2 | 1 |
| | Improvement after 8 weeks | 6 | 2 | 4 | 3 | 3 | 4 | 4 |
| | No change | 4 | 6 | 6 | 7 | 6 | 6 | 5 |
| | Dis-continuation of treatment due to exacerbation | 0 | 2 | 0 | 0 | 1 | 0 | 1 |
| | Total | 10 | 10 | 10 | 10 | 10 | 10 | 10 |

Table 13

| Psoriasis disease improvement test | | Test Examples 16 | 17 | 18 | 19 | 20 | 21 | 22 |
|---|---|---|---|---|---|---|---|---|
| Ointment used | | Comp. Ex.1 | Comp. Ex.2 | Comp. Ex.3 | Comp. Ex.4 | Comp. Ex.5 | Comp. Ex.6 | Comp. Ex.7 |
| Evaluation (No.of persons) | Improvement after 4 weeks | 2 | 1 | 2 | 2 | 1 | 0 | 1 |
| | Improvement after 8 weeks | 7 | 1 | 5 | 4 | 4 | 2 | 5 |
| | No change | 3 | 7 | 5 | 6 | 5 | 7 | 5 |
| | Dis-continuation of treatment due to exacerbation | 0 | 2 | 0 | 0 | 1 | 1 | 0 |
| | Total | 10 | 10 | 10 | 10 | 10 | 10 | 10 |

Test Examples 23 and 24

Ointments prepared in Examples 1, 7 and 13 as skin external preparations were tested on 30 patients diagnosed as atopic dermatitis in accordance with the following test method.

(Test method 2)

The ointments were applied to the affected parts of the subjects two to three times per day for eight consecutive weeks. After 2, 4, 6, and 8 weeks, the degree of improvement of erythema, papule, squama, pruritus, tylosis, crust, or erosio was observed by visual inspection for evaluation. The symptoms of the skin were categorized into erythema, papule, squama, pruritus, tylosis, crust, or erosio, and the evaluation was made by score change before and after the ointment application. The degree of symptom of each category was scored as follows: 3 points for severe degree, 2 points for medium degree, 1 point for slight degree, and 0 point for no symptom. The number of points is the mean value of the skin symptoms of 10 subjects. The results are shown in Tables 14 to 16.

Table 14

| | | Test Example 23 | | | | |
|---|---|---|---|---|---|---|
| Ointment used | | Ointment prepared in Example 1 | | | | |
| Application period (week) | | 0 | 2 | 4 | 6 | 8 |
| Evaluation average | Erythema | 3.0 | 0.6 | 0.1 | 0.0 | 0.0 |
| | Papule | 3.0 | 1.2 | 0.2 | 0.1 | 0.0 |
| | Squama | 3.0 | 1.0 | 0.2 | 0.2 | 0.0 |
| | Pruritus | 3.0 | 1.2 | 0.1 | 0.1 | 0.0 |
| | Tylosis | 3.0 | 1.8 | 1.2 | 1.0 | 0.0 |
| | Crust | 3.0 | 0.3 | 0.2 | 0.1 | 0.0 |
| | Erosio | 3.0 | 1.2 | 0.4 | 0.1 | 0.0 |

Table 15

| | | Test Example 24 | | | | |
|---|---|---|---|---|---|---|
| Ointment used | | Ointment prepared in Example 7 | | | | |
| Application period (week) | | 0 | 2 | 4 | 6 | 8 |
| Evaluation average | Erythema | 3.0 | 0.6 | 0.2 | 0.1 | 0.0 |
| | Papule | 3.0 | 1.2 | 0.1 | 0.1 | 0.0 |
| | Squama | 3.0 | 0.8 | 0.1 | 0.0 | 0.0 |
| | Pruritus | 3.0 | 0.8 | 0.1 | 0.0 | 0.0 |
| | Tylosis | 3.0 | 1.8 | 1.0 | 1.0 | 0.0 |
| | Crust | 3.0 | 1.0 | 0.1 | 0.1 | 0.0 |
| | Erosio | 3.0 | 0.8 | 0.2 | 0.1 | 0.0 |

Table 16

| | | Test Example 25 | | | | |
|---|---|---|---|---|---|---|
| Ointment used | | Ointment prepared in Example 13 | | | | |
| Application period (week) | | 0 | 2 | 4 | 6 | 8 |
| Evaluation average | Erythema | 3.0 | 0.8 | 0.4 | 0.2 | 0.0 |
| | Papule | 3.0 | 1.2 | 0.4 | 0.1 | 0.0 |
| | Squama | 3.0 | 1.0 | 0.2 | 0.0 | 0.0 |
| | Pruritus | 3.0 | 1.0 | 0.2 | 0.0 | 0.0 |
| | Tylosis | 3.0 | 1.2 | 1.0 | 0.2 | 0.0 |
| | Crust | 3.0 | 1.0 | 0.1 | 0.1 | 0.0 |
| | Erosio | 3.0 | 0.8 | 0.1 | 0.0 | 0.0 |

Example 19

A cream 1 was prepared in accordance with the following formulation. White petrolatum, Stearyl alcohol, and POE(60) hardened castor oil were taken in prescribed amounts, heated to 70 °C in a water bath, and homogenized by stirring. Subsequently, a liver oil to which BHA had previously been added in a prescribed amount was added thereto.

On the other hand, propylene glycol and PC-3 were taken in prescribed amounts, and heated to 70 °C. Subsequently, methyl paraoxybenzoate and propyl paraoxybenzoate were added and mixed in prescribed amounts. Further, purified water was added in a prescribed amount thereto, and mixed to be an emulsion by means of a homomixer. The resulting emulsion and the previously prepared mixture containing a liver oil and white petrolatum were mixed, and emulsified by means of a paddle mixer. Then, the resulting emulsion was cooled, and well mixed until the oil component is hardened, to prepare a cream.

| Formulation of cream 1 | |
|---|---|
| Blending composition | Blending amount |
| Liver oil | 5.0 g |
| BHA | 5 mg |
| Polymer PC-4 | 0.1 g |

(continued)

| Formulation of cream 1 | |
|---|---|
| Blending composition | Blending amount |
| White petrolatum | 19.2 g |
| Stearyl alcohol | 15.4 g |
| Propylene glycol | 9.2 g |
| POE(60) hardened castor oil | 3.1 g |
| Glycerin monostearate | 0.7 g |
| Methyl paraoxybenzoate | 77 mg |
| Propyl paraoxybenzoate | 77 mg |
| Purified water | Remainder |
| Total amount | 100 g |

Example 20

Each preparation was prepared in accordance with its corresponding formulation shown below with the method in accordance with Example 19.

| Formulation of cream 2 | |
|---|---|
| Blending composition | Blending amount |
| Perilla oil | 7.0 g |
| Bufexamac | 3.0 g |
| BHT | 5 mg |
| Polymer PC-5 | 0.1 g |
| White petrolatum | 30.8 g |
| Cetanol | 7.9 g |
| White beeswax | 3.9 g |
| Sorbitan sesquioleate | 4.1 g |
| Lauromacrogol | 0.38 g |
| Ethyl paraoxybenzoate | 77 mg |
| Butyl paraoxybenzoate | 77 mg |
| Purified water | Remainder |
| Total amount | 100 g |

| Formulation of cream 3 | |
|---|---|
| Blending composition | Blending amount |
| Linseed oil | 4.0 g |
| Vitamin A palmitate | 0.1 g |
| Polymer PC-6 | 0.1 g |
| BHT | 5 mg |
| White petrolatum | 19.2 g |
| Stearyl alcohol | 15.4 g |
| Propylene alcohol | 9.2 g |
| POE(60) hardened castor oil | 3.1 g |
| Glycerin monostearate | 0.7 g |
| Ethyl paraoxybenzoate | 77 mg |
| Butyl paraoxybenzoate | 77 mg |
| Purified water | Reminder |

(continued)

| Formulation of cream 3 | |
|---|---|
| Blending composition | Blending amount |
| Total amount | 100 g |

| Formulation of oleaginous ointment | |
|---|---|
| Blending composition | Blending amount |
| Tunny fish oil | 5.0 g |
| BHA | 5 mg |
| Polymer PC-7 | 0.1 g |
| Zinc oxide | 15.9 g |
| Liquid paraffin | 8.0 g |
| White ointment | Reminder |
| Total amount | 100 g |

| Formulation of cream 4 | |
|---|---|
| Blending composition | Blending amount |
| Perilla frutescens oil | 5.0 g |
| BHT | 5 mg |
| Polymer PC-1 | 0.1 g |
| White petrolatum | 19.2 g |
| Stearyl alcohol | 15.4 g |
| Propylene alcohol | 9.2 g |
| POE(60) hardened castor oil | 3.1 g |
| Glycerin monostearate | 0.7 g |
| Methyl paraoxybenzoate | 0.77 g |
| Propyl paraoxybenzoate | 0.77 g |
| Ascorbic palmitate | 0.65 g |
| Purified water | Remainder |
| Total amount | 100 g |

| Formulation of lotion | |
|---|---|
| Blending composition | Blending amount |
| Linseed oil | 5.0 g |
| BHT | 5 mg |
| Polymer PC-2 | 0.1 g |
| Octyl dodecyl myristate | 3.4 g |
| POE(60) hardened castor oil | 3.1 g |
| Glycerin monostearate | 2.2 g |
| Methyl paraoxybenzoate | 0.25 g |
| Propyl paraoxybenzoate | 0.75 g |
| Propylene glycol | 5.0 g |
| Sodium pyrrolidone carboxylate | 1.1 g |
| Perfume | 0.01 g |
| Purified water | Remainder |

(continued)

| Formulation of lotion | |
|---|---|
| Blending composition | Blending amount |
| Total amount | 100 g |

| Formulation of plaster preparation | |
|---|---|
| Blending composition | Blending amount |
| Liver oil | 5.0 g |
| BHT | 5 mg |
| Polymer PC-3 | 0.1 mg |
| Diethyl sebacate | 3.2 g |
| Sorbitan sesquioleate | 1.4 g |
| POE (20) sorbitan monooleate | 0.5 g |
| Sodium polyacrylate | 5.1 g |
| Sodium carboxymethyl cellulose | 2.7 g |
| Dried aluminium hydroxide gel | 0.1 g |
| Sodium edetate | 0.05 g |
| Titanium dioxide | 1.2 g |
| Glycerin | 10.0 g |
| Gelatin | 2.2 g |
| Peppermint oil | 0.1 g |
| Purified water | Remainder |
| Total amount | 100 g |

As a result, products of Comparative Examples 1 to 7 did not exhibit any synergistic effect on the treatment of dermatitises such as skin roughening and dermatitis (psoriasis disease), whereas products of Examples 2 to 18 containing the components A to C of the present invention exhibited the synergistic effects thereon. Further, the tests with products of Examples 1, 7, and 13 indicated that, by mixing the components A to C of the present invention therein, it was possible to obtain remarkable effects on atopic dermatitis.

Still further, it has been indicated that it is possible to form the composition of the present invention in a preparation as an external preparation such as cream, oleaginous ointment, plaster preparation, or lotion in accordance with Examples 19 to 25.

## Claims

1. A skin external preparation composition, comprising: a natural oil and/or fat (A) containing an ω-3 unsaturated fatty acid and an ω-6 unsaturated fatty acid, the content of the ω-3 unsaturated fatty acid being 4 times or more the content of the ω-6 unsaturated fatty acid; a phosphorylcholine-like group containing polymer (B); and an antioxidant (C).

2. The skin external preparation composition according to claim 1, wherein the blending ratio of the natural oil and/or fat (A) is from 90 to 99.999 % by weight based on the total amount of the natural oil and/or fat (A) and the phosphorylcholine-like group containing polymer (B), the blending ratio of the phosphorylcholine-like group containing polymer (B) is from 10 to 0.01 % by weight based on the total amount of the natural oil and/or fat (A) and the phosphorylcholine-like group containing polymer (B), and the blending ratio of the antioxidant (c) is from 0.001 to 1 part by weight based on a total amount of 100 parts by weight of the natural oil and/or fat (A) and the phosphorylcholine-like group containing polymer (B).

3. The skin external preparation composition according to claim 1, wherein the phosphorylcholine-like group containing polymer (B) is a polymer obtained by polymerizing a monomer composition containing a 2-(meth)acryloyloxyethyl phosphorylcholine monomer, and has a weight average molecular weight of from 5000 to 5000000.

**4.** The skin external preparation composition according to claim 1, wherein the fatty acid composition of the natural oil and/or fat (A) contains 10 to 40 % by weight of a docosahexaenoic acid, 5 to 20 % by weight of an eicosapentaenoic acid, and 0 to 5 % by weight of an arachidonic acid, and the content of the docosahexaenoic acid is greater than the content of the eicosapentaenoic acid.

**5.** The skin external preparation composition according to claim 4, wherein the fatty acid composition of the natural oil and/or fat (A) further contains 50 to 70 % by weight of an $\alpha$-linolenic acid, and 0 to 20 % by weight of a linolic acid.

**6.** The skin external preparation composition according to any one of claim 1, 4, or 5, wherein the natural oil and/or fat (A) is selected from the group consisting of a liver oil, a tunny fish oil, a perilla oil, a perilla frutescens oil, and a linseed oil, and mixtures thereof.

**7.** The skin external preparation composition according to any one of claim 1, 4, or 5, wherein the peroxide value of the natural oil and/or fat (A) is from 0 to 30 meq/kg, and the aldehyde value thereof is from 0 to 0.035 mmol/g.

**8.** The skin external preparation composition according to claim 1, wherein the antioxidant (C) is selected from the group consisting of vitamin C, dibutylhydroxytoluene, butylhydroxyanisole, citric acid, and mixtures thereof.

## INTERNATIONAL SEARCH REPORT

International application No.
PCT/JP99/02883

A. CLASSIFICATION OF SUBJECT MATTER
Int.Cl$^6$ A61K31/20, A61K47/10, A61K47/30, A61K7/48, A61K35/60, A61K35/78

According to International Patent Classification (IPC) or to both national classification and IPC

B. FIELDS SEARCHED

Minimum documentation searched (classification system followed by classification symbols)
Int.Cl$^6$ A61K31/20, A61K47/10, A61K47/30, A61K7/48, A61K35/60, A61K35/78

Documentation searched other than minimum documentation to the extent that such documents are included in the fields searched

Electronic data base consulted during the international search (name of data base and, where practicable, search terms used)
CA (STN), MEDLINE (STN)

C. DOCUMENTS CONSIDERED TO BE RELEVANT

| Category* | Citation of document, with indication, where appropriate, of the relevant passages | Relevant to claim No. |
|---|---|---|
| Y | JP, 8-175988, A (PROSA BV), 9 July, 1996 (09. 07. 96) & EP, 699437, A1 & US, 5776978, A | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 August, 1999 (23. 08. 99) | 31 August, 1999 (31. 08. 99) |
| Name and mailing address of the ISA/ Japanese Patent Office | Authorized officer |
| Facsimile No. | Telephone No. |

Form PCT/ISA/210 (second sheet) (July 1992)